# EUROPÄISCHE PATENTSCHRIFT

(11) **EP 1 593 370 B1**
(45) Veröffentlichungstag und Bekanntmachung des Hinweises auf die Patenterteilung: **09.01.2013**
(21) Anmeldenummer: 05008975.4
(22) Anmeldetag: 23.04.2005
(51) Int. Cl.: A61K 8/34, A61K 8/39, A61Q 15/00, A61Q 1/06, A61Q 17/04, A61Q 19/00, A61Q 17/00

(54) **Glycerinethergemisch, dieses enthaltende kosmetische Zusammensetzung sowie Verfahren zu dessen Herstellung**
Glycerin ether mixture, a composition comprising glycerin ether mixture and a process for making the same
Mélange d'éthers de glycérine, une composition contenant celui-ci et un procédé pour la fabriquer

(30) Priorität: 04.05.2004 DE 102004022252
(43) Veröffentlichungstag der Anmeldung: 09.11.2005
(73) Patentinhaber: Cognis IP Management GmbH, 40589 Düsseldorf (DE)
(72) Erfinder: Brüning, Stefan, Philadelphia PA 19118 (US); Ansmann, Achim, 40699 Erkrath (DE); Gondek, Helga, 40589 Düsseldorf (DE); Schmid, Karl Heinz, 40822 Mettmann (DE); Neuss, Michael, 50997 Köln (DE); Albers, Thomas, 40597 Düsseldorf (DE)

(56) Entgegenhaltungen:
- EP-A- 0 599 433
- WO-A-00/67713
- WO-A-03/040072
- WO-A-03/055559
- DE-A1- 3 943 070
- PATENT ABSTRACTS OF JAPAN Bd. 2002, Nr. 10, 10. Oktober 2002 (2002-10-10) & JP 2002 161018 A (NIKKO SEIYAKU KK), 4. Juni 2002 (2002-06-04)

## Beschreibung

### Gebiet der Erfindung

Gegenstand der Erfindung sind ein Glycerinethergemisch, ein Verfahren zu dessen Herstellung sowie kosmetische Zusammensetzungen, die dieses Glycerinethergemisch enthalten.

### Stand der Technik

Die Verwendung von Glycerinmonoalkylethern in kosmetischen Zusammensetzungen ist grundsätzlich bereits bekannt. So beschreibt die EP 0 599 433 A1 Glycerinmonoalkylether mit einer C₆-C₁₈- und vorzugsweise einer C₆-C₁₂-Alkylgruppe als desodorierenden Wirkstoff in wässrigen oder alkoholischen Deolösungen. In der DE 100 47 759 A1 werden C₆-C₁₈-Glycerinmonoalkylether als antimikrobielle Wirkstoffe in Feuchttüchern eingesetzt. Auch in diesem Fall sind die kürzerkettigen Glycerinmonoalkylether bevorzugt.

Aufgrund ihrer Ether-Struktur sind Partial-Glycerinether stabil gegen hydrolytische oder katalytische Abbaureaktionen durch saure Antiperspirant-Salze (Aluminium- bzw. Aluminium/Zirkoniumsalze), was ein sehr wichtiges Kriterium für den Rohstoffeinsatz im Antiperspirant-Bereich ist.

Als Emulgatoren oder Konsistenzgeber für kosmetische Zusammensetzungen haben Glycerinether bislang wenig Beachtung gefunden. In wasserhaltigen Antiperspirant/Deo-Emulsionen kommen üblicherweise Fettalkohole wie Cetearyl-, Stearyl- und Behenylalkohol, Fettsäureester, aber auch Triglyceride wie Glycerinmonostearat als Co-Emulgatoren bzw. als konsistenzgebende Komponenten zum Einsatz. Fettalkohole und Hydroxyfettsäuren sind aufgrund ihrer chemischen Struktur stabil, Fettsäureester, aber auch Triglyceride wie Glycerinmonostearat hingegen nicht.
Die anwendungstechnische Entwicklung stabiler und viskositätsstabiler, aber auch sensorisch angenehmer Emulsionen stellt besonders im Bereich Antiperspirant/Deo an den Entwickler eine hohe Anforderung. Gerade der hohe Salzgehalt, der niedrige pH-Bereich und die Tatsache, dass die in Wasser gelösten Antiperspirant-Wirkstoffe deutlich klebende Endeigenschaften in der Formulierung verursachen, erfordern neue Rohstoff-Lösungen auf dem Gebiet der Antiperspirant/Deo-Wachse, um verbesserte Antiperspirant/Deo-Formulierungen zu entwickeln.

Im Bereich wasserfreier Antiperspirant-Stift- sowie Antiperspirant-Soft Solid-Formulierungen finden Fettalkohole wie Cetearyl-, Stearyl- und Behenylalkohol, aber auch Hydroxyfettsäuren wie 12-Hydroxystearinsäure in Kombination mit dem Polymer N-Acylsäureamid häufigen Einsatz (vgl. US 5,429,816 A). Derartige Systeme führen bei Anwendung auf den aufgetragenen Hautpartien nicht immer zu einem optimalen Hautgefühl, selbst bei Auswahl optimierter Emollients.

Aufgabe der Erfindung ist es entsprechend, einen über einen breiten Anwendungsbereich einsetzbaren Emulgator bzw. Konsistenzgeber anzugeben, der im Bereich von wässrigen O/W-Emulsionen bis hin zu festen kosmetischen Zusammensetzungen und insbesondere in Antiperspirant/Deo-Formulierungen zu stabilen und sensorisch als angenehm empfundenen Produkten führt.

### Beschreibung der Erfindung

Die Lösung dieser Ausgabe gelingt mit dem Glycerinethergemisch gemäß Anspruch 1, das Bestandteil der kosmetischen Zusammensetzung gemäß Anspruch 7 ist. Die Erfindung betrifft weiterhin ein Verfahren zur Herstellung des Glycerinethergemisches gemäß Anspruch 13. Bevorzugte Weiterbildungen sind in den jeweiligen Unteransprüchen beschrieben.

In einem ersten Aspekt betrifft die Erfindung also ein Glycerinethergemisch, welches im Wesentlichen besteht aus
a) wenigstens einem C₁₂₋₂₂-Glycerinmonoalkylether
b) wenigstens einem C₁₂₋₂₂-Glycerindialkylether
   und gegebenenfalls
c) wenigstens einem C₁₂₋₂₂-Glycerintrialkylether und/oder
d) wenigstens einem C₁₂₋₂₂-Fettalkohol,
wobei die Komponenten a) und b) zusammen mindestens 50 Gew.-% des Glycerinethergemisches ausmachen und das Gewichtsverhältnis von Komponente a) zu Komponente b) im Bereich von 3 : 1 bis 1 : 2 liegt.

Überraschenderweise wurde nun gefunden, dass ein zumindest aus Mono- und Dialkylethern des Glycerins bestehendes Gemisch mit dem oben angegebenen bestimmten Gewichtsverhältnis zwischen Mono- und Dialkylether außerordentlich gute emulgierende und konsistenzgebende Eigenschaften aufweist. Diese Eigenschaften übertreffen diejenigen der jeweils für sich allein eingesetzten Mono- oder Dialkylether des Glycerins. Besonders vorteilhafte Eigenschaften werden erzielt, wenn das Gewichtsverhältnis von Komponente a) (C₁₂₋₂₂-Glycerinmonoalkylether) zu Komponente b) (C₁₂₋₂₂-Glycerindialkylether) im Bereich von 1 : 1 bis 1,3 : 1 liegt

Die erfindungsgemäße Glycerinether-Zusammensetzung kann lediglich aus den Komponenten a) und b) in dem angegebenen Mischungsverhältnis bestehen. Es können jedoch zusätzlich, in der Regel bedingt durch das Herstellungsverfahren, C₁₂₋₂₂-Glycerintrialkylether (Komponente c)) und/oder C₁₂₋₂₂-Fettalkohol (Komponente d)) im Gemisch vorhanden sein. Der überwiegende Anteil des Glycerinethergemisches besteht jedoch aus den Komponenten a) und b), die gemeinsam mindestens 50 Gew.-%, insbesondere mehr als 60 Gew.% und besonders bevorzugt mehr als 70 Gew.-%, des Gemisches ausmachen. Neben den Komponenten a) bis d) können weitere Komponenten in untergeordneten Mengen in der Zusammensetzung enthalten sein. Diese weiteren Komponenten können beispielsweise ebenfalls durch das Herstellungsverfahren bedingt in das Gemisch gelangen. Typische Begleitsubstanzen sind beispielsweise Wasserreste und rohstoffbedingte Verunreinigungen. Ihr Anteil im beanspruchten Glycerinethergemisch liegt bei maximal 10 Gew.-%, vorzugsweise bei höchstens 5 Gew.-%. Entsprechend bedeutet im Rahmen dieser Erfindung "im Wesentlichen besteht aus", dass das beanspruchte Glycerinethergemisch zu mindestens 90 Gew.% aus den Komponenten a) und b) sowie, falls vorhanden, Komponenten c) und/oder d) besteht.

Bei dem C₁₂₋₂₂-Alkylrest der Komponenten a) bis d) kann es sich um einen gesättigten oder ungesättigten, verzweigten oder unverzweigten Alkylrest handeln. Geeignete Reste sind zum Beispiel Cetyl-, Palmoleyl-, Stearyl-, Isostearyl-, Oleyl-, Elaidyl-, Petroselinyl-, Linolyl-, Linolenyl-, Elaeostearyl-, Arachyl-, Gadoleyl-, Behenyl-, Erucyl- und Brassidyl-. Es können auch deren technische Mischungen vorliegen, wie sie z.B. ausgehend von natürlichen Fetten und Ölen anfallen.

Besonders bevorzugt werden Alkylreste mit 16 bis 18 Kohlenstoffatomen eingesetzt und unter diesen besonders die gesättigten und unverzweigten.

Grundsätzlich ist es möglich, jede der einzelnen Komponenten des erfindungsgemäßen Glycerinethergemisches gesondert herzustellen und die einzelnen Komponenten in einem geeigneten Mischungsverhältnis miteinander zu vermischen. In solchen Fällen können sich die Alkylreste der einzelnen Komponenten voneinander unterschieden. Bevorzugt ist es jedoch, wenn Alkyl für alle Komponenten a) bis d) gleich ist. Die Herstellung erfolgt dann bevorzugt so, dass aus der Umsetzung direkt das Gemisch der Komponenten a) und b) sowie gegebenenfalls c) und d) in der gewünschten Konzentrationsverteilung erhalten wird.

Besonders geeignet als (Co)-Emulgator oder Konsistenzgeber in kosmetischen Zusammensetzungen ist ein Glycerinethergemisch, das im Wesentlichen besteht aus 35 - 45 Gew.% der Komponente a), 25 - 45 Gew.-% der Komponente b), 1 - 5 Gew.-% der Komponente c) und 8 - 27 Gew.-% der Komponente d). Noch bevorzugter ist ein Glycerinethergemisch, das im Wesentlichen besteht aus 40 Gew.% der Komponente a), 30 - 40 Gew.% der Komponente b), 2 - 3 Gew.-% der Komponente c) und 13 - 22 Gew.% der Komponente d).

Wie bereits erwähnt, erfolgt die Herstellung des erfindungsgemäßen Glycerinethergemisches bevorzugt so, dass die Mischung mit den gewünschten Mengen der Einzelkomponenten als Umsetzungsprodukt der Synthese erhalten wird. Besonders gut eignet sich eine Abwandlung des von der Anmelderin in der WO 03/040072 A1 beschriebenen Verfahrens. Dort zielt die Synthese hauptsächlich auf die Bildung von Monoalkylethem ab, während die Dialkylether mit in der Regel nicht mehr als 10 Gew.-% und maximal 16 Gew.-% anfallen. Um das Verhältnis von Mono- zu Dialkylether in den erfindungsgemäß gewünschten Bereich zu bringen und den Anteil an höher veretherten Produkten zu steigern, wird der Anteil an Glycerin in den Edukten im Vergleich zu den in der WO 03/040072 A1 beschriebenen Beispielen herabgesetzt.

Im erfindungsgemäßen Verfahren zur Herstellung des Glycerinethergemisches wird also, in Analogie zum Verfahren der WO 03/040072 A1, Glycerin mit einer Base deprotoniert und das entstehende Wasser kontinuierlich aus der Reaktionsmischung entfernt, das deprotonierte Glycerin mit einer Komponente Z), die ausgewählt ist aus einem Alkylsulfat oder einem Schwefelsäurealkylester, umgesetzt, und die gebildete feste Phase und die wässrige Phase werden vom entstandenen Glycerinethergemisch abgetrennt. Im Unterschied zur WO 03/040072 A1 wird jedoch das Glycerin in einem molaren Verhältnis zur Komponente Z) eingesetzt, das im Bereich von 4 : 1 bis 2 : 1 liegt.
Für den Erhalt der erfindungsgemäß besonders bevorzugten Glycerinethergemische eignet sich besonders ein Verhältnis von Glycerin zu Komponente Z) im Bereich von etwa 3 : 1.

Für alle sonstigen Verfahrensparameter, Aufarbeitungs- und Reinigungsschritte usw. kann auf die WO 03/040072 A1 verwiesen werden.

In einem weiteren Aspekt betrifft die Erfindung eine kosmetische Zusammensetzung, die das erfindungsgemäße Glycerinethergemisch enthält und zwar insbesondere als Emulgator und/oder Konsistenzgeber.

Die Art der kosmetischen Zusammensetzung ist nicht besonders beschränkt. Bei den in dem erfindungsgemäßen Gemisch enthaltenen Glycerinethern handelt es sich um feste, wachsartige Verbindungen. Das Glycerinethergemisch kann daher in alle kosmetischen Zusammensetzungen inkorporiert werden, die bereits bisher Wachse und hier vor allem Wachse mit emulgierenden oder konsistenzgebenden Eigenschaften enthielten. Geeignete kosmetische Zusammensetzungen reichen daher über wasserfreie feste Formulierungen wie zum Beispiel Stiftpräparate (Deostifte, Lippenstifte etc.) und halbfeste Formulierungen (so genannte "Soft solids", wie sie unter anderem in Deopräparaten Anwendung finden) bis hin zu wässrigen Emulsionen (O/W-Emulsionen für Cremes, Lotionen, Deo-Roll-ons usw.).

Das erfindungsgemäße Glycerinethergemisch eignet sich besonders als Emulgator oder Konsistenzgeber in kosmetischen Zusammensetzungen. Die ansonsten in den kosmetischen Zusammensetzungen vorhandenen Komponenten wie Emollients (Ölkörper, Fette, Wachse), kosmetische oder pharmazeutische Wirkstoffe, Verdickungsmittel, Überfettungsmittel, Füllstoffe, Farbstoffe, Pigmente, Stabilisatoren (Antioxidantien, Konservierungsmittel), UV-Lichtschutzfilter, Filmbildner, Quellmittel, Hydrotrope und Parfumöle können grundsätzlich wie bisher verwendet werden. Auf sie muss daher hier nicht näher eingegangen werden.

Besonders günstige Ergebnisse werden erzielt, wenn das Glycerinethergemisch in der kosmetischen Zusammensetzung in einem Anteil von 0,2 bis 20 Gew.-% und insbesondere 0,5 bis 5 Gew.% enthalten ist.

Während das Glycerinethergemisch grundsätzlich als alleiniger Emulgator oder Konsistenzgeber verwendet werden kann, enthält eine bevorzugte kosmetische Zusammensetzung das Glycerinethergemisch in Kombination mit wenigstens einem weiteren Emulgator und/oder Konsistenzgeber. Die Menge des Glycerinethergemisches hängt dabei von den anderen verwendeten Komponenten ab. Grundsätzlich beträgt aber der Anteil des Glycerinethergemisches in der Gesamtmenge aller Emulgatoren und Konsistenzgeber zweckmäßig 5 bis 90 Gew.-%, insbesondere 10 bis 80 Gew.-% und bevorzugt 15 bis 75 Gew.-%.

Besonders gute konsistenzgebende Eigenschaften für die kosmetische Zusammensetzung werden erreicht, wenn das erfindungsgemäße Glycerinethergemisch in Abmischung mit Triglyceriden wie hydriertem Rizinusöl, mit Fettalkoholen wie Stearylalkohol oder Behenylalkohl oder mit Hydroxyfettsäuren wie 12-Hydroxystearinsäure verwendet wird. Dabei zeigen gerade die C₁₆-C₂₂-Partialglycerinether einen hohen konsistenzgebenden Einfluss auf unterschiedlichste Emollients und sind somit höchst geeignet für wasserfreie Antiperspirant-Soft solid- sowie Antiperspirant-Stift-Formulierungen.

Über die reinen konsistenzgebenden Eigenschaften hinaus werden mit den erfindungsgemäßen Glycerinethergemischen äußerst homogene innere Strukturen sowie Oberflächen-Strukturen in der kosmetischen Formulierung erzielt. In dem immer häufiger vom Markt geforderten hohen sensorischen Anspruch an Antiperspirant-Produkte liefert gerade diese neue Glycerinethermischung einen stark verbesserten Einfluss auf den sensorischen Gesamteindruck der wasserfreien Endformulierung. So wird eine höhere Hautglätte und Hautweichheit erzielt, was zu einem deutlich erhöhten Pflegeeindruck der Gesamtformulierung führt.

So zeigen diese Wachse auch in wässrigen Antiperspirant/Deo-Emulsionen sehr gute konsistenzgebende Eigenschaften durch den Aufbau lamellarer Phasen, die sich als äußerst stabil erweisen. Neben verbesserten Emulsionsstabilitäten werden auch erhöhte Viskositätsstabilitäten während der Lagerung erzielt. Aufgrund der chemischen Struktur dieser Partialglyceride und dem damit einhergehenden lamellaren Strukturaufbau wird ein äußerst pflegender, aber auch sensorisch leichter Gesamteindruck der Formulierungen auf der Haut des Anwenders erzielt. Auch in PIT-Emulsionen zeigen die Partialglyceride sehr hohe (co-)emulgierende Eigenschaften und liefern damit sehr stabile, dünnflüssige Emulsionen.

Eine bevorzugte Anwendung der Erfindung liegt daher auf dem Gebiet der Antiperspirant-Formulierungen. Eine besonders bevorzugte kosmetische Zusammensetzung zeichnet sich deshalb dadurch aus, dass sie wenigstens eine desodorierende, adstringierende oder antimikrobielle Verbindung enthält.

Die Erfindung soll nachfolgend anhand von Beispielen näher erläutert werden.

### Beispiele

### Beispiel 1

### Herstellung eines erfindungsgemäßen Glycerinethergemisches

In einen 2-I-Vierhalskolben werden 276 g (3 Mol) Glycerin auf 120 °C erwärmt, langsam 100 g (1,25 Mol) 50%ige Natronlauge zugetropft und dabei kontinuierlich das entstehende Wasser bei 120 °C und einem Vakuum von 100 mbar abkondensiert. Zum Schluss der Wasserabscheidung wird das Vakuum auf 10 mbar reduziert. Das so gebildete Na-Glycerinat wird mit 380 g (1 Mol) Lanette-E-Pulver (Natriumcetylstearylsulfat-Pulver) versetzt, suspendiert und bei 180°C 8 h lang gerührt. Die Reaktionskontrolle erfolgt über die Bestimmung des Anionentensidgehaltes, der nach 8 Stunden Reaktionszeit deutlich unterhalb von 1 % liegt. Zur Aufarbeitung wird die Reaktionsmischung mit 225 ml Wasser und 5 ml 50 %iger Natronlauge bei einer Temperatur von 90 °C gemischt und anschließend die Phasentrennungen abgewartet. Anschließend werden die entstandenen Phasen getrennt. Dazu wird die untere Phase zur Abtrennung des ausgefällten Natriumsulfates filtriert und die obere organische Phase (Glycerinetherphase) mit 250 ml Wasser einer Temperatur von 90 °C gewaschen. Abermals werden die Phasen getrennt. Mittels Vakuumdestillation wird die organische Phase destillativ vom Wasser befreit. Das C_{16/18}-Glycerinethergemisch verbleibt im Destillationsrückstand. Das Produkt enthält ca. 40 Gew.-% Mono-C_{16/18}-glycerinether, ca. 40 Gew.-% Di-C_{16/18}-glycerinether, ca. 2 Gew.-% Tri-C_{16/18}-glycerinether und ca. 15 Gew.-% C_{16/18}-Fettalkohol.

### Vergleichsbeispiel 1

### Herstellung eines Glycerinethergemisches zu Vergleichszwecken

Entsprechend Beispiel 1 wurde unter Verwendung von 0,5 Mol anstelle 3 Mol Glycerin ein Glycerinethergemisch hergestellt, das Mono- zu Diether des Glycerins im Verhältnis 1 : 4 enthielt.

### Formulierungsbeispiele

### Herstellung kosmetischer Zusammensetzungen

Unter Verwendung des in Beispiel 1 hergestellten Glycerinethergemisches wurden verschiedene kosmetische Zusammensetzungen hergestellt. Zusätzlich wurden mit dem Glycerinethergemisch zu Vergleichszwecken gemäß Vergleichsbeispiel 1, dem entsprechenden Monoglycerinether und anderen üblichen Emulgatoren bzw. Konsistenzgebem nicht erfindungsgemäße Vergleichsformulierungen hergestellt.

Die Zusammensetzungen der kosmetischen Zubereitungen sind in nachfolgenden Tabellen 1 bis 5 angegeben. Dabei enthält Tabelle 1 Formulierungsbeispiele für Antiperspirant-Cremes und -Roll-ons, Tabelle 2 solche für Pflegecremes, Tabelle 3 solche für Lippenstifte, Tabelle 4 solche für Sonnencremes und Körper- und Gesichtspflegecremes und Tabelle 5 schließlich solche für Antiperspirant-Stifte und -Soft solids. Die Mengen der Komponenten sind in Gew.-% angegeben. Die Vergleichsbeispiele sind in den Tabellen jeweils mit "V..." bezeichnet, die erfindungsgemäßen Beispiele mit "E...".

Einige der Formulierungen wurden auf ihre Eigenschaften hin untersucht.
Die Viskositätsmessungen wurden für die Formulierungen der Tabelle 1 mit einem Viskosimeter Brookfield RVF, Spindel 5, 10 UpM bestimmt, diejenigen in Tabelle 2 mit einem Viskosimeter Brookfield RVT, Spindel TE, 4 UpM mit Helipath.
Die Härtemessungen wurden mit einem Penetrometer Petrotest PNR 10 (Petrotest Instruments GmbH & Co. KG) durchgeführt (Mikrokonus: 5.0 g; Fallstab: 47.5 g; Messtemperatur: 23 °C; Härte = mm Eindringtiefe in 5 Sekunden).

Die sensorischen Untersuchungen wurden von jeweils 4 geschulten Testpersonen durchgeführt. Hierzu wurden die Formulierungen jeweils auf den Unterarm der Testperson aufgetragen, und folgende Kriterien wurden nach einer Skala von - 2 bis + 2 bewertet: Struktur (-2: sehr inhomogen bis +2: sehr homogen), Viskostabilität und Stabilität (-2: gering bis +2: hoch), Sensorik (-2: schlecht bis +2: sehr gut) und Ölaustritt (-2: stark bis +2: keiner). Die Ergebnisse sind als Mittelwerte der individuellen Beurteilungen in der nachfolgenden Tabelle zusammengestellt.

**Tabelle 1**

| Antiperspirant-Cremes und -Roll-ons | | | | | | | | | | | | | | |
|---|---|---|---|---|---|---|---|---|---|---|---|---|---|---|
| ***Bestandteile*** | ***INCI*** | ***V1*** | ***V2*** | ***V3*** | ***V4*** | ***V5*** | ***V6₋*** | ***V7*** | ***V8*** | ***E1*** | ***E2*** | ***E3*** | ***E4*** | ***E5*** |
| Eumulgin B2 | Ceteareth-20 | 2.0 | 2.0 | 2.0 | 2.0 | 2.0 | 2.0 | 2.0 | 2.0 | 2.0 | 2.0 | 2.0 | 2.0 | 1.0 |
| Eumulgin B3 | Ceteareth-30 | - | - | - | - | - | - | - | - | - | - | - | - | 1.0 |
| Eumulgin S2 | Steareth-2 | - | - | - | - | - | - | - | - | - | - | - | - | 1.0 |
| Lanette O | Cetearyl Alcohol | 5.0 | 5.0 | - | - | - | - | - | - | - | - | 1.0 | - | - |
| Lanette 22 | Behenyl Alcohol | - | - | - | - | - | - | - | - | - | - | - | - | - |
| Cutina MD | Glycerylstearate | - | - | 5.0 | 5.0 | - | - | - | - | - | - | - | 1.0 | - |
| C16/18-Monoglycerinether | | - | - | - | - | 5.0 | 5.0 | - | - | - | - | - | - | - |
| Glycerinethergemisch | Vqlbsp. 1 Mono/Di (1:4) | - | - | - | - | - | - | 5.0 | 5.0 | - | - | - | - | - |
| Glycerinethergemisch | Bsp.1 Mono/Di (1:1) | - | - | - | - | - | - | - | - | 5.0 | 5.0 | 4.0 | 4.0 | 2.5 |
| Cetiol S | Diethylhexylcyclohexane | 16.0 | - | 16.0 | - | 16.0 | - | 16.0 | - | 16.0 | - | 2.0 | 2.0 | 8.0 |
| Eutanol G16S | Hexyldecyl Stearate | - | 16.0 | | 16.0 | - | 16.0 | - | 16.0 | - | 16.0 | 2.0 | 2.0 | 2.0 |
| Cetiol OE | Dicaprylyl Ether | - | - | - | - | - | - | - | - | - | - | 4.0 | 4.0 | - |
| Cetiol CC | Dicaprylyl Carbonat | - | - | - | - | - | - | - | - | - | - | 4.0 | 4.0 | 2.0 |
| Eutanol G | Octyldodecanol | - | - | - | - | - | - | - | - | - | - | 2.0 | 2.0 | - |
| DC 245 | Cyclomethicone | - | - | - | - | - | - | - | - | - | - | 2.0 | 2.0 | 2.0 |
| Hydagen CAT | Triethylcitrate | - | - | - | - | - | - | - | - | - | - | - | - | 1.0 |
| Triclosan | Triclosan | - | - | - | - | - | - | - | - | - | - | - | - | 0.1 |
| Locron L | Aluminum Chlorohydrate | 40.0 | 40.0 | 40.0 | 40.0 | 40.0 | 40.0 | 40.0 | 40.0 | 40.0 | 40.0 | 40.0 | 40.0 | |
| Rezal 36 GC | Aluminum Zirconium Chlorohydrate | - | - | - | - | - | - | - | - | - | - | - | - | 40.0 |
| Glycerin | Glycerine | 3.0 | 3.0 | 3.0 | 3.0 | 3.0 | 3.0 | 3.0 | 3.0 | 3.0 | 3.0 | 3.0 | 3.0 | 3.0 |
| Hydagen DCMF | Chitosan | - | - | - | - | - | - | - | - | - | - | - | - | 0.05 |
| Water | Aqua | add 100 | add 100 | add 100 | add 100 | add 100 | add 100 | add 100 | add 100 | add 100 | add 100 | add 100 | add 100 | add 100 |
| Preservation | | qs | qs | qs | qs | qs | qs | qs | qs | qs | qs | qs | qs | qs |
| *Viskosität* | [mPas] | 15.000 | 20.000 | 7.000 | 9.000 | 1.000 | 1.000 | 15.000 | 12.000 | 40.000 | 40.000 | 35.000 | 35.000 | 5.000 |
| *Struktur* | | +1 | +1 | +1 | +1 | 0 | 0 | +1 | +1 | +2 | +2 | +2 | +2 | +2 |
| *Viskostabilität* | | +1 | +1 | -1 | -1 | -2 | -2 | -1 | -1 | +2 | +2 | +2 | +2 | +2 |
| *Stabilität* | | +1 | +1 | -2 | -2 | -2 | -2 | -1 | -1 | +2 | +2 | +2 | +2 | +2 |
| *Sensorik* | | +1 | +1 | +1 | +1 | - | - | - | - | +2 | +2 | +2 | +2 | +2 |

**Tabelle 2**

| Pflegecremes | | | | | | |
|---|---|---|---|---|---|---|
| ***Bestandteile*** | ***INCI*** | ***V 9*** | ***V 10*** | ***V 11*** | ***V 12*** | ***E 7*** |
| Emulgade PL 68/50 | Cetearyl Glucoside, Cetearyl Alcohol | 2.7 | 2.7 | 2.7 | 2.7 | 2.7 |
| Lanette O | Cetearyl Alcohol | 1.6 | - | - | - | - |
| Cutina MD | Glycerylstearate | - | 1.6 | - | - | - |
| C16/18-Monoglycerinether | | - | - | 1.6 | - | - |
| Glycerinethergemisch Vqlbsp. 1 Mono/Di (1:4) | | - | - | - | 1.6 | - |
| Glycerinethergemisch Bsp.1 Mono/Di(1:1) | | - | - | - | - | 1.6 |
| Myritol 331 | Cocoglycerides | 4.0 | 4.0 | 4.0 | 4.0 | 4.0 |
| Cetiol OE | Dicaprylyl Ether | 2.0 | 2.0 | 2.0 | 2.0 | 2.0 |
| Cetiol V | Decyl Oleate | 2.0 | 2.0 | 2.0 | 2.0 | 2.0 |
| Dimethicone DC 200 | Dimethicone | 0.5 | 0.5 | 0.5 | 0.5 | 0.5 |
| Cetiol J600 | Oleyl Erucate | 3.0 | 3.0 | 3.0 | 3.0 | 3.0 |
| Glycerin | Glycerine | 3.0 | 3.0 | 3.0 | 3.0 | 3.0 |
| Water | Aqua | add 100 | add 100 | add 100 | add 100 | add 100 |
| Preservation | | qs | qs | qs | qs | qs |
| *Viskosität [mPas]* | | 50.000 | 60.000 | < 5.000 | < 10.000 | 140.000 |
| *Struktur* | | +1 | +1 | +1 | +1 | +2 |
| *Viskostabilität* | | -2 | -1 | -2 | -2 | +2 |
| *Stabilität* | | +1 | +1 | -2 | -2 | +2 |
| *Sensorik* | | +1 | 0 | - | - | +2 |

**Tabelle 3**

| Lippenstift | | |
|---|---|---|
| ***Bestandteile*** | ***INCI*** | ***E 8*** |
| Myritol 318 | Caprylic/Capric Triglyceride | 14,0 |
| Myritol PC | Propylene Glycol Dicaprylate/Dicaprate | 6,0 |
| Eutanol G | Octyldodecanol | 17,0 |
| Bienenwachs 8100 (Carl&Co) | Cera Alba | 5,0 |
| Canelilla Wax | Candelina cera | 5,0 |
| Carnauba Wax | Carnauba cera | 7,0 |
| Monomuls 90 L 12 | Glyceryl Laurate | 3,0 |
| Dehymuls PGPH | Polyglyceryl 2 Dipolyhydroxystearate | 4,0 |
| Glycerinethergemisch Bsp. 1 | | 8,0 |
| Castor Oil | Hydrogenated Castor Oil | 10,0 |
| Copherol F 1300 | Tocopherol | 2,0 |
| Hydagen CMF | Chitosan Glycolate | 10,0 |
| Farbpigmente | | nach Bedarf |

**Tabelle 4**

| Sonnencremes und Körper- und Gesichtspflegecremes | | | | | | | |
|---|---|---|---|---|---|---|---|
| ***Bestandteile*** | ***INCI*** | ***E9*** | ***E10*** | ***E11*** | ***E12*** | ***E13*** | ***E14*** |
| Emulgade PL 6850 | Cetearyl Glucoside, Cetearyl Alcohol | 4,5 | - | - | - | - | 5,0 |
| Amphisol K | Potassium Cetyl Phosphate | 1,0 | - | - | - | - | 0,5 |
| CuGna GMS | Glyceryl Stearate | - | - | - | - | - | 0,5 |
| Lanette O | Cetearyl Alkohol | - | - | - | - | 2,5 | - |
| Glycerinetherqemisch Bsp. 1 | | 1,0 | 1,0 | 1,5 | 3,0 | 3,0 | 3,0 |
| Eumulgin VL 75 | Lauryl Glucoside, Polyglyceryl-2 Dipolyhydroxystearate, Glycerin | - | 4,0 | 4,0 | - | 4,0 | - |
| Lanette E | Sodium Cetearyl Sulfate | - | 1,0 | 1,0 | - | - | - |
| Emulgade SE-PF | Glyceryl Stearate, Ceteareth-20, Ceteareth-12, Cetearyl Alcohol, Cetyl Palmitate | - | - | - | 5,0 | - | - |
| Eumulgin B1 | Ceteareth-12 | - | - | - | 1,0 | - | - |
| Dimethicone DC 200 | Dimethicone | - | - | - | 0,5 | 0,5 | 0,5 |
| Cetiol OE | Dicaprylyl Ether | 2,0 | - | - | 2,0 | | |
| Cetiol CC | Dicaprylyl Carbonate | - | - | 12,0 | - | 4,0 | 5,0 |
| Cetiol J600 | Oleyl Erucate | - | - | - | - | 2,0 | - |
| Cetiol S | Diethylhexylcyclohexane | - | - | - | 1,0 | - | - |
| Myritol331 | Cocoglycerides | 8,0 | 12,0 | 10,0 | - | 2,0 | - |
| Cetiol B | Dibutyl Adipate | - | 10,0 | - | - | - | - |
| Cetiol 868 | Ethylhexyl Stearate | - | - | - | - | - | 4,0 |
| Eutanol G16 | Hexyldecanol | 3,0 | - | - | - | - | - |
| Copherol 1300 | Tocopherol | 1,0 | - | - | - | 1,0 | 1,0 |
| Cetiol SN | Cetearyl Isononanoate | - | - | - | 2,0 | - | - |
| Cegesoft PFO | Passionflora Incarnata (EU), Passiflora Incarnata Seed Oil (non EU) | - | - | - | - | - | 2,0 |
| Cegesoft PS6 | Olus (EU), Vegetable Oil (non EU) | - | - | - | - | - | 3,0 |
| Dow Coming 200 Fluid | Dimethicone | - | - | - | 2,0 | 0,5 | - |
| Neo Heliopan, Type 303 | Octocrylene | 7,0 | 9,0 | 9,0 | - | - | - |
| Neo Heliopan, Type 357 | Butyl Methoxydibenzoylmethane | - | - | - | 1,2 | - | - |
| Neo Heliopan, Type MBC | 4-Methylbenzylidene Camphor | 3,0 | - | - | 1,0 | - | - |
| Neo Heliopan AV | Ethylhexyl Methoxycinnamate | - | 7,5 | 7,5 | 3,0 | - | - |
| Zinkoxid NDM | Zinc Oxide | 7,0 | 5,0 | - | - | - | - |
| Eusolex T 2000 | Titanium Dioxide, Alumina, Simethicone | - | - | 5,0 | - | - | - |
| Veegum Ultra | Magnesium Aluminium Silicates | 1,5 | 1,5 | 1,5 | - | - | - |
| Keltrol T | Xanthan Gum | 0,5 | 0,5 | 0,5 | - | - | - |
| Hispagel 200 | Glycerin, Glyceryl Polyacrylate | | - | - | 5,0 | - | - |
| Carbopol 980 (2%) | Polyacrylate | - | - | - | - | 15,0 | - |
| SFE 839 (GE Bayer Silicones) | Cyclopentasiloxane, Dimethicone/Vinyldimethicone Crosspolymer | - | - | - | - | 5,0 | - |
| Sepigel 305 (Seppic) | Polyacrylamide, C13-14 Isoparaffine, Laureth-7 | - | - | - | 1,0 | - | - |
| Dry Flo Plus (Starch and Chemical Limited) | Aluminium Starch Octenylsuccinate | - | - | - | - | 5,0 | - |
| Hydagen B | Bisabolol | - | 0,05 | 0,5 | - | - | - |
| 1,3 Butylene Glycol | Butylene Glycol | - | - | - | - | - | 2,0 |
| Glycerin | Glycerin | 3,0 | 3,0 | 3,0 | - | 2,0 | 2,0 |
| KOH (20%ig) | Potassium Hvdroxide | - | - | - | - | 0,5 | - |
| Wasser, Konservierungsmittel | | Add 100 | Add 100 | Add 100 | Add 100 | Add 100 | Add 100 |

**Tabelle 5**

| Antiperspirant-Stifte und -Soft solids | | | | | | | | |
|---|---|---|---|---|---|---|---|---|
| ***Bestandteile*** | ***INCl*** | ***V13*** | ***V14*** | ***V15*** | ***E15*** | ***E16*** | ***E17*** | ***E18*** |
| Lanette 18 | Stearyl Alcohol | 20.0 | 16.0 | - | | | 4.0 | - |
| Cutina HR | Hydrogenated Castor Oil | - | 4.0 | - | - | 4.0 | 2.0 | - |
| | 12-Hydroxystearic Acid | - | - | 12.0 | - | - | - | 8.0 |
| | GP1 | - | - | - | - | - | - | - |
| Glycerinethergemisch Bsp. 1 | | - | - | - | 20.0 | 16.0 | 8.0 | 4.0 |
| DC 245 | Cyclomethicone | 33.0 | 33.0 | 37.0 | 33.0 | 33.0 | 35.0 | 37.0 |
| Cetiol OE | Dicaprylyl Ether | 9.0 | 9.0 | 10.0 | 9.0 | 9.0 | 10.0 | 10.0 |
| Cetiol S | Diethylhexylcyclohexane | 15.0 | 10.0 | 18.0 | 10.0 | 15.0 | 18.0 | 18.0 |
| Arlamol E | PPG15 Stearyether | | 2.5 | | 2.5 | | | |
| DC 200 | Dimethicone | | 2.5 | | 2.5 | | | |
| Rezal 36 GP | Aluminum Zirconium Tetrachlorohydrex GLY | 23.0 | 23.0 | 23.0 | - | - | 23.0 | - |
| Locron P | Aluminum Chlorohydrate | - | - | - | 23.0 | 23.0 | - | 23.0 |
| *Härte* (*Penetration*) | | 4.2 | 4.2 | 4.6 | 5.0 | 5.0 | 9.0 | 4.0 |
| *Struktur* | | 0 | 0 | +1 | +1 | +1 | +2 | +2 |
| *Stabilität* | | -1 | +2 | +1 | +2 | +2 | +2 | +2 |
| *Ölaustritt* | | -1 | +1 | +1 | +1 | +1 | +1 | +1 |
| *Sensorik* | | +1 | +1 | +1 | +2 | +2 | +2 | +2 |

## Patentansprüche

1. Glycerinethergemisch, welches zu mindestens 90 gew% aus den Komponenten a) und b) sowie, falls vorhanden, Komponenten c) und d) besteht, wobei
a) wenigstens einem C₁₂₋₂₂-Glycerinmonoalkylether,
b) wenigstens einem C₁₂₋₂₂-Glycerindialkylether,
und gegebenenfalls
c) wenigstens einem C₁₂₋₂₂-Glycerintrialkylether und/oder
d) wenigstens einem C₁₂₋₂₂-Fettalkohol, ist
**dadurch gekennzeichnet, dass** die Komponenten a) und b) zusammen mindestens 50 Gew.-% des Glycerinethergemisches ausmachen und das Gewichtsverhältnis von Komponente a) zu Komponente b) im Bereich von 3 : 1 bis 1 : 2 liegt.

2. Glycerinethergemisch gemäß Anspruch 1,
**dadurch gekennzeichnet, dass** das Gewichtsverhältnis von Komponente a) zu Komponente b) im Bereich von 1 : 1 bis 1,3 : 1 liegt.

3. Glycerinethergemisch gemäß Anspruch 1 oder 2,
**dadurch gekennzeichnet, dass** Alkyl für einen gesättigten oder ungesättigten, verzweigten oder unverzweigten, insbesondere einen gesättigten und unverzweigten, Alkylrest mit 16 bis 18 Kohlenstoffatomen steht.

4. Glycerinethergemisch gemäß einem der Ansprüche 1 bis 3,
**dadurch gekennzeichnet, dass** Alkyl für alle Komponenten a) bis d) gleich ist.

5. Glycerinethergemisch gemäß einem der Ansprüche 1 bis 4,
**dadurch gekennzeichnet, dass** es besteht aus
35 - 45 Gew.-% der Komponente a),
25 - 45 Gew.-% der Komponente b),
1 - 5 Gew.-% der Komponente c) und
8 - 27 Gew.-% der Komponente d).

6. Glycerinethergemisch gemäß Anspruch 3 oder 4,
**dadurch gekennzeichnet, dass** es besteht aus
40 Gew.-% der Komponente a),
30 - 40 Gew.-% der Komponente b),
2 - 3 Gew.-% der Komponente c) und
13 - 22 Gew.-% der Komponente d).

7. Kosmetische Zusammensetzung,
**dadurch gekennzeichnet, dass** sie das Glycerinethergemisch gemäß einem der Ansprüche 1 bis 6 enthält.

8. Kosmetische Zusammensetzung gemäß Anspruch 7,
**dadurch gekennzeichnet dass** sie das Glycerinethergemisch als Emulgator und/oder Konsistenzgeber enthält.

9. Kosmetische Zusammensetzung gemäß Anspruch 7 oder 8,
**dadurch gekennzeichnet, dass** sie das Glycerinethergemisch in einem Anteil von 0,2 bis 20 Gew.-% und insbesondere 0,5 bis 5 Gew.-% enthält.

10. Kosmetische Zusammensetzung gemäß einem der Ansprüche 7 bis 9,
**dadurch gekennzeichnet, dass** sie das Glycerinethergemisch in Kombination mit wenigstens einem weiteren Emulgator und/oder Konsistenzgeber enthält.

11. Kosmetische Zusammensetzung gemäß Anspruch 9,
**dadurch gekennzeichnet, dass** der Anteil des Glycerinethergemisches in der Gesamtmenge aller Emulgatoren und Konsistenzgeber 5 bis 90 Gew.-%, insbesondere 10 bis 80 Gew.-% und bevorzugt 15 bis 75 Gew.-%, beträgt.

12. Kosmetische Zusammensetzung gemäß einem der Ansprüche 7 bis 10,
**dadurch gekennzeichnet, dass** sie wenigstens eine desodorierende, adstringierende oder antimikrobielle Verbindung enthält.

13. Verfahren zur Herstellung des Glycerinethergemisches gemäß einem der Ansprüche 1 bis 6,
worin Glycerin mit einer Base deprotoniert und das entstehende Wasser kontinuierlich aus der Reaktionsmischung entfernt wird, das deprotonierte Glycerin mit einer Komponente Z), die ausgewählt ist aus einem Alkylsulfat oder einem Schwefelsäurealkylester, umgesetzt wird, wobei Alkyl die in Ansprüchen 1, 3 oder 4 angegebene Bedeutung hat, und die gebildete feste Phase und die wässrige Phase vom entstandenen Glycerinethergemisch abgetrennt werden, **dadurch gekennzeichnet, dass** das Glycerin in einem molaren Verhältnis zur Komponente Z) eingesetzt wird, das im Bereich von 4 : 1 bis 2 : 1 und insbesondere im Bereich von etwa 3 : 1 liegt.

## Claims

1. Glycerol ether mixture which consists, to at least 90% by weight, of the components a) and b) and also, if present, components c) and d), wherein
a) is at least one C₁₂₋₂₂-glycerol monoalkyl ether,
b) is at least one C₁₂₋₂₂-glycerol dialkyl ether, and optionally
c) is at least one C₁₂₋₂₂-glycerol trialkyl ether and/or
d) is at least one C₁₂₋₂₂-fatty alcohol,
**characterized in that** the components a) and b) together constitute at least 50% by weight of the glycerol ether mixture and the weight ratio of component a) to component b) is in the range from 3:1 to 1:2.

2. Glycerol ether mixture according to Claim 1,
**characterized in that** the weight ratio of component a) to component b) is in the range from 1:1 to 1.3:1.

3. Glycerol ether mixture according to Claim 1 or 2,
**characterized in that** alkyl is a saturated or unsaturated, branched or unbranched, in particular a saturated and unbranched, alkyl radical having 16 to 18 carbon atoms.

4. Glycerol ether mixture according to one of Claims 1 to 3,
**characterized in that** alkyl is identical for all components a) to d).

5. Glycerol ether mixture according to one of Claims 1 to 4,
**characterized in that** it consists of
35-45% by weight of component a),
25-45% by weight of component b),
1-5% by weight of component c) and
8-27% by weight of component d).

6. Glycerol ether mixture according to Claim 3 or 4,
**characterized in that** it consists of
40% by weight of component a),
30-40% by weight of component b),
2-3% by weight of component c) and
13-22% by weight of component d).

7. Cosmetic composition,
**characterized in that** it comprises the glycerol ether mixture according to one of Claims 1 to 6.

8. Cosmetic composition according to Claim 7,
**characterized in that** it comprises the glycerol ether mixture as emulsifier and/or consistency regulator.

9. Cosmetic composition according to Claim 7 or 8,
**characterized in that** it comprises the glycerol ether mixture in a fraction of from 0.2 to 20% by weight and in particular 0.5 to 5% by weight.

10. Cosmetic composition according to one of Claims 7 to 9,
**characterized in that** it comprises the glycerol ether mixture in combination with at least one further emulsifier and/or consistency regulator.

11. Cosmetic composition according to Claim 9,
**characterized in that** the fraction of the glycerol ether mixture in the total amount of all emulsifiers and consistency regulators is 5 to 90% by weight, in particular 10 to 80% by weight and preferably 15 to 75% by weight.

12. Cosmetic composition according to one of Claims 7 to 10,
**characterized in that** it comprises at least one deodorizing, astringent or antimicrobial compound.

13. Process for the preparation of the glycerol ether mixture according to one of Claims 1 to 6,
in which glycerol is deprotonated with a base and the resulting water is removed continuously from the reaction mixture, the deprotonated glycerol is reacted with a component Z), which is selected from an alkyl sulphate or a sulphuric acid alkyl ester, wherein alkyl has the meaning given in Claim 1, 3 or 4, and the formed solid phase and the aqueous phase are separated from the resulting glycerol ether mixture, **characterized in that** the glycerol is used in a molar ratio relative to the component Z) which is in the range from 4:1 to 2:1 and in particular in the region of about 3:1.

## Revendications

1. Mélange d'éthers de glycérol, qui consiste en au moins 90 % en poids des composants a) et b) ainsi que, s'ils sont présents, des composants c) et d),
a) étant au moins un éther monoalkylique de glycérol en C₁₂-C₂₂,
b) étant au moins un éther dialkylique de glycérol en C₁₂-C₂₂,
et éventuellement
c) étant au moins un éther trialkylique de glycérol en C₁₂-C₂₂ et/ou
d) étant au moins un alcool gras en C₁₂-C₂₂,
**caractérisé en ce que** les composants a) et b) constituent ensemble au moins 50 % en poids du mélange d'éthers de glycérol et le rapport pondéral du composant a) au composant b) se situe dans la plage allant de 3 : 1 à 1 : 2.

2. Mélange d'éthers de glycérol selon la revendication 1,
**caractérisé en ce que** le rapport pondéral du composant a) au composant b) se situe dans la plage allant de 1 : 1 à 1,3 : 1.

3. Mélange d'éthers de glycérol selon la revendication 1 ou 2,
**caractérisé en ce qu'**alkyle représente un radical alkyle saturé ou insaturé, ramifié ou non ramifié, en particulier un radical alkyle saturé et non ramifié ayant de 16 à 18 atomes de carbone.

4. Mélange d'éthers de glycérol selon l'une quelconque des revendications 1 à 3,
**caractérisé en ce que** le radical alkyle est le même pour tous les composants a) à d).

5. Mélange d'éthers de glycérol selon l'une quelconque des revendications 1 à 4,
**caractérisé en ce qu'**il consiste en
35 - 45 % en poids du composant a),
25 - 45 % en poids du composant b),
1 - 5 % en poids du composant c) et
8 - 27 % en poids du composant d).

6. Mélange d'éthers de glycérol selon la revendication 3 ou 4,
**caractérisé en ce qu'**il consiste en
40 % en poids du composant a),
30 - 40 % en poids du composant b),
2 - 3 % en poids du composant c) et
13 - 22 % en poids du composant d).

7. Composition cosmétique,
**caractérisée en ce qu'**elle contient le mélange d'éthers de glycérol selon l'une quelconque des revendications 1 à 6.

8. Composition cosmétique selon la revendication 7,
**caractérisée en ce qu'**elle contient le mélange d'éthers de glycérol en tant qu'émulsifiant et/ou agent de consistance.

9. Composition cosmétique selon la revendication 7 ou 8,
**caractérisée en ce qu'**elle contient le mélange d'éthers de glycérol en une proportion de 0,2 à 20 % en poids et en particulier de 0,5 à 5 % en poids.

10. Composition cosmétique selon l'une quelconque des revendications 7 à 9,
**caractérisée en ce qu'**elle contient le mélange d'éthers de glycérol en association avec au moins un autre émulsifiant et/ou agent de consistance.

11. Composition cosmétique selon la revendication 9,
**caractérisée en ce que** la proportion du mélange d'éthers de glycérol dans la quantité totale de tous les émulsifiants et agents de consistance vaut de 5 à 90 % en poids, en particulier de 10 à 80 % en poids, et de préférence de 15 à 75 % en poids.

12. Composition cosmétique selon l'une quelconque des revendications 7 à 10,
**caractérisée en ce qu'**elle contient au moins un composé déodorant, astringent ou antimicrobien.

13. Procédé pour la préparation du mélange d'éthers de glycérol selon l'une quelconque des revendications 1 à 6,
dans lequel on soumet du glycérol à une déprotonation à l'aide d'une base et on élimine en continu du mélange réactionnel l'eau formée, on fait réagir le glycérol déprotoné avec un composant Z), qui est choisi parmi un alkylsulfate ou un sulfate d'alkyle, alkyle ayant la signification indiquée dans les revendications 1, 3 ou 4, et on sépare la phase solide formée et la phase aqueuse du mélange d'éthers de glycérol résultant, **caractérisé en ce qu'**on utilise le glycérol en un rapport molaire au composant Z) qui se situe dans la plage allant de 4 : 1 à 2 : 1 et en particulier dans la plage d'environ 3 : 1.
